# EUROPÄISCHE PATENTSCHRIFT

(11) **EP 0 308 792 B1**
(45) Veröffentlichungstag und Bekanntmachung des Hinweises auf die Patenterteilung: **28.07.1993**
(21) Anmeldenummer: 88114993.4
(22) Anmeldetag: 14.09.1988
(51) Int. Cl.: C07D 491/04, C07D 491/10, A61K 31/435

(54) **Azachromanderivate**
Azachromane derivatives
Dérivés d'azachromane

(30) Priorität: 24.09.1987 DE 3732146
(43) Veröffentlichungstag der Anmeldung: 29.03.1989
(73) Patentinhaber: MERCK PATENT GmbH, D-64271 Darmstadt (DE)
(72) Erfinder: Baumgarth, Manfred, Dr., D-6100 Darmstadt (DE); Gericke, Rolf, Dr., D-6104 Seeheim (DE); Lues, Ingeborg, Dr., D-6100 Darmstadt (DE); De Peyer, Jacques, Dr., D-6104 Seeheim (DE); Bergmann, Rolf, Dr., D-6101 Reichelsheim (DE)

(56) Entgegenhaltungen:
- EP-A- 0 205 292
- EP-A- 0 273 262
- WO-A-87/07607

## Beschreibung

Die Erfindung betrifft Azachromanderivate der Formel I 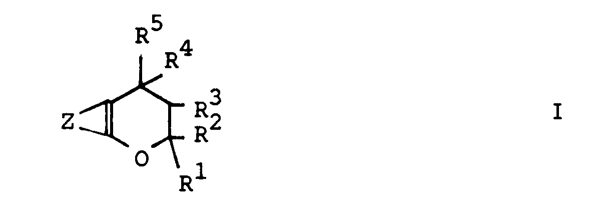
worin
- Z: (a) -N=CH-CH=CH-,
(b) -CH=N-CH=CH-,
(c) -CH=CH-N=CH- oder
(d) -CH=CH-CH=N-,
- R¹: A,
- R²: H oder A,
- R¹ und R²: zusammen auch Alkylen mit 3-6 C-Atomen,
- R³: OH oder OAc,
- R⁴: H,
- R³ und R⁴: zusammen auch eine Bindung,
- R⁵: einen unsubstituierten oder ein- oder zweifach durch A, F, Cl, Br, J, OH, OA, OAc, NO₂, NH₂, AcNH, HOOC und/oder AOOC substituierten 1H-2-Pyridon-1-yl-, 1H-6-Pyridazinon-1-yl-, 1H-2-Pyrimidinon-1-yl-, 1H-6-Pyrimidinon-1-yl-, 1H-2-Pyrazinon-1-yl- oder 1H-2-Thiopyridon-1-yl-rest, wobei diese Reste auch partiell hydriert sein können,
- A: Alkyl mit 1-6 C-Atomen und
- Ac: Alkanoyl mit 1-8 C-Atomen oder Aroyl mit 7-11 C-Atomen
bedeuten,
sowie deren Salze.

Ähnliche Verbindungen sind bekannt aus der EP-A 1-205292.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z. B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Die Verbindungen der Formel I umschließen:
a) die 5-Azachromanderivate der Formel Ia (= I, worin Z -N=CH-C=CH- bedeutet; 2H-Pyrano[3,2-b]pyridine und deren 3,4-Dihydroderivate);
b) die 6-Azachromanderivate der Formel Ib (= I, worin Z -CH=N-CH=CH- bedeutet; 2H-Pyrano[3,2-c]pyridine und deren 3,4-Dihydroderivate; Ring-Index Nr. 1698);
c) die 7-Azachromanderivate der Formel Ic (= I, worin Z -CH=CH-N=CH- bedeutet; 2H-Pyrano[2,3-c]pyridine und deren 3,4-Dihydroderivate);
d) die 8-Azachromanderivate der Formel Ic (= I, worin Z -CH=CH-CH=N- bedeutet; 2H-Pyrano[2,3-d]pyridine und deren 3,4-Dihydroderivate; Ring-Index Nr. 1701).

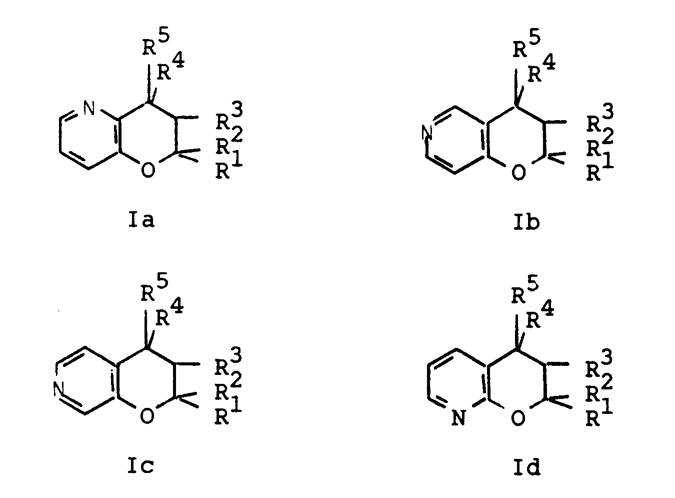

Bevorzugt sind die Verbindungen der Formel Ib.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt -(CH₂)ₙ-, wobei n 3, 4, 5 oder 6 bedeutet.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl.

R³ und R⁴ sind bevorzugt zusammen eine Bindung. Falls R⁴ H bedeutet, ist R³ bevorzugt OH, O-CHO oder O-COCH₃.

R⁵ ist bevorzugt unsubstituiertes 1H-2-Pyridon-1-yl, 1H-2-Pyrazinon-1-yl oder 1H-4-Hydroxy-2-pyridon-1-yl, ferner bevorzugt unsubstituiertes 1H-6-Pyridazinon-1-yl, 4,5-Dihydro-1H-6-pyridazinon-1-yl, 1H-2-Pyrimidinon-1-yl, 1H-6-Pyrimidinon-1-yl oder 1H-2-Thiopyridon-1-yl. Falls R⁵ einen substituierten Pyridon- bzw. Thiopyridonring bedeutet, so ist dieser Ring vorzugsweise einfach in 3-, 4- oder 5-Stellung oder zweifach in 3- und 5-Stellung substituiert. Besonders bevorzugte Substituenten sind OH, NO₂ und NH₂, ferner AOOC, OA, Cl, Br und NHCOCH₃, besonders bevorzugte substituierte Reste R⁵ im einzelnen 4-, ferner 3-, 5- und 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3- oder 5-Amino-, 3- oder 5-Carboxy-, 3- oder 5-Methoxycarbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido- und 3-Acetamido-5-brom-1H-2-pyridon-1-yl bzw. -1H-2-thiopyridon-1-yl, 1H-3-, 1H-4- oder 1H-5-Hydroxy-6-pyridazinon-1-yl, 1H-3-, 1H-4-oder 1H-5- Ethoxycarbonyl-6-pyridazinon-1-yl, 1H-4-, 1H-5- oder 1H-6-Hydroxy-2-pyrimidinon-1-yl, 1H-2- oder 1H-4-Hydroxy-6-pyrimidinon-1-yl.

R⁵ kann ferner bevorzugt bedeuten: 3,4-Dihydro-1H-2-pyridon-1-yl, 2,3-Dihydro-6H-2-pyridon-1-yl, 5,6-Dihydro-1H-2-pyridon-1-yl, 2,3-Dihydro-1H-6-pyridazinon-1-yl, 1,2-Dihydro-5H-6-pyridazinon-1-yl, 3,4-Dihydro-1H-2-pyrimidinon-1-yl, 1,6-Dihydro-3H-2-pyrimidinon-1-yl, 5,6-Dihydro-1H-2-pyrimidinon-1-yl, 2,3-Dihydro-1H-6-pyrimidinon-1-yl, 1,2-Dihydro-5H-6-pyrimidinon-1-yl, 4,5-Dihydro-1H-6-pyrimidinon-1-yl, 3,4-Dihydro-1H-2-pyrazinon-1-yl, 1,6-Dihydro-3H-2-pyrazinon-1-yl, 5,6-Dihydro-1H-2-pyrazinon-1-yl, 3,4-Dihydro-1H-2-thiopyridon-1-yl, 2,3-Dihydro-6H-2-thiopyridon-1-yl, 5,6-Dihydro-1H-2-thiopyridon-1-yl.

Dementsprechend sind Gegenstand der Erfindung insbesondere diejenigen Verbindungen der Formeln I, Ia, Ib, Ic und Id, in denen mindestens einer der genannten Reste eine der vorstehend angegebenen bevorzugten Bedeutungen hat. Einige bevorzugte Gruppen von Verbindungen können durch die nachstehenden Formeln Ie bis Im sowie Iae bis Iam, Ibe bis Ibm, Ice bis Icm und Ide bis Idm ausgedruckt werden, die den Formeln I, Ia, Ib, Ic und Id entsprechen und worin die nicht näher bezeichneten Reste die bei diesen Formeln angegebene Bedeutung haben, worin jedoch
- in Ie, Iae, Ibe, Ice und Ide: R¹ und R² jeweils A bedeuten;
- in If, Iaf, Ibf, Icf und Idf: R¹ und R² jeweils CH₃ bedeuten;
- in Ig, Iag, Ibg, Icg und Idg: R¹ und R² zusammen Alkylen mit 3-6 C-Atomen bedeuten;
- in Ih, Iah, Ibh, Ich und Idh: R⁵ 1H-2-Pyridon-1-yl, 1H-2-Pyrazinon-1-yl, 1H-6-Pyridazinon-1-yl, 4,5-Dihydro-1H-6-pyridazinon-1-yl, 1H-2-Pyrimidinon-1-yl, 1H-6-Pyrimidinon-1-yl, 1H-2-Thiopyridon- 1-yl, 3-, 4-, 5-oder 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3- oder 5-Amino-, 3- oder 5-Carboxy-, 3- oder 5-Methoxycarbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido-oder 3-Acetamido-5-brom-1H-2-pyridon-1-yl oder -1H-2-thiopyridon-1-yl, 1H-3-, 1H-4- oder 1H-5-Hydroxy-6-pyridazinon-1-yl, 1H-3-, 1H-4- oder 1H-5-Ethoxycarbonyl-6-pyridazinon-1-yl, 1H-4-, 1H-5- oder 1H-6-Hydroxy-2-pyrimidinon-1-yl, 1H-2- oder 1H-4-Hydroxy-6-pyrimidinon-1-yl bedeutet;
- in Ii, Iai, Ibi, Ici und Idi: R⁵ 1H-2-Pyridon-1-yl, 1H-2-Pyrazinon-1-yl oder 1H-4-Hydroxy-2-pyridon-1-yl bedeutet;
- In Ij, Iaj, Ibj, Icj und Idj: R⁵ 1H-2-Pyridon-1-yl bedeutet;
- in Ik, Iak, Ibk, Ick und Idk: R¹ und
R² jeweils CH₃ und
R⁵ 1H-2-Pyridon-1-yl, 1H-2-Pyrazinon-1-yl, 1H-6-Pyridazinon-1-yl, 4,5-Dihydro-1H-6-pyridazinon-1-yl, 1H-2-Pyrimidinon-1-yl, 1H-6-Pyrimidinon-1-yl, 1H-2-Thiopyridon-1-yl, 3-, 4-, 5-oder 6-Hydroxy-, 3-, 4-, 5- oder 6-Methoxy-, 3-, 4-, 5- oder 6-Acetoxy-, 3-, 5- oder 6-Chlor-, 3- oder 5-Nitro-, 3- oder 5-Amino-, 3- oder 5-Carboxy-, 3- oder 5-Methoxycarbonyl-, 3- oder 5-Ethoxycarbonyl-, 3- oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-,3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido- oder 3-Acetamido-5-brom-1H-2-pyridon-1-yl oder -1H-2-thiopyridon-1-yl, 1H-3-, 1H-4- oder 1H-5-Hydroxy-6-pyridazinon-1-yl, 1H-3-, 1H-4- oder 1H-5-Ethoxycarbonyl-6-pyridazinon-1-yl, 1H-4-, 1H-5- oder 1H-6-Hydroxy-2-pyrimidinon-1-yl, 1H-2- oder 1H-4-Hydroxy-6-pyrimidinon-1-yl bedeuten;
- in Il, Ial, Ibl, Icl und Idl: R¹ und
R² jeweils CH₃ und
R⁵ 1H-2-Pyridon-1-yl, 1H-2-Pyrazinon-1-yl oder 1H-4-Hydroxy-2-pyridon-1-yl bedeuten;
- in Im, Iam, Ibm, Icm und Idm: R¹ und
R² jeweils CH₃ und
R⁵ 1H-2-Pyridon-1-yl bedeuten.

Weiterhin sind bevorzugt Verbindungen der Formeln I′ sowie Ia′ bis Im′, Iae′ bis Iam′, Ibe′ bis Ibm′, Ice′ bis Icm′ sowie Ide′ bis Idm', die den Formeln I sowie Ia bis Im, Iae bis Iam, Ibe bis Ibm, Ice bis Icm, sowie Ide bis Idm entsprechen, worin jedoch jeweils zusätzlich R³ OH, OCHO oder OCOCH₃ und R⁴ H bedeuten.

Weiterhin sind bevorzugt Verbindungen der Formeln I'' sowie Ia′′ bis Im′′, Iae′′ bis Iam′′, Ibe′′ bis Ibm′′, Ice′′ bis Icm′′, Ide′′ bis Idm′′, die den Formeln I sowie Ia bis Im, Iae bis Iam, Ibe bis Ibm, Ice bis Icm sowie Ide bis Idm entsprechen, worin jedoch jeweils zusätzlich R³ und R⁴ zusammen eine Bindung bedeuten.

Im übrigen haben vor- und nachstehend die Reste R¹ bis R⁵, A und Ac die bei Formel I angegebenen Bedeutungen, wenn nicht ausdrücklich etwas anderes angegeben ist.

Gegenstand der Erfindung ist ferner ein Verfahren zur Herstellung von Azachromanderivaten der Formel I, dadurch gekennzeichent, daß man ein 3,4-Epoxy-aza-chroman der Formel II 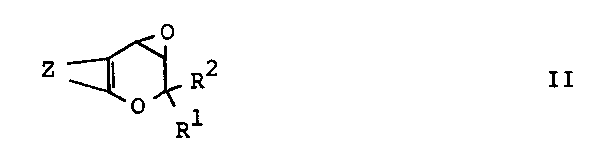
worin
R¹, R² und Z die bei Formel I angegebene Bedeutung haben,
mit einer Verbindung der Formel III

R⁵-H III

worin R⁵ die bei Formel I angegebene Bedeutung hat oder mit einem ihrer reaktionsfähigen Derivate umsetzt oder daß man ein sonst der Formel I entsprechendes N-Oxid reduziert
und/oder daß man eine Verbindung der Formel I, worin R³ OH und R⁴ H bedeutet, dehydratisiert und/oder daß man in einer Verbindung der Formel I einen oder mehrere der Reste R³ und/oder R⁵ in andere Reste R³ und/oder R⁵ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Die Ausgangsstoffe II sind teilweise, diejenigen der Formel III sind in der Regel bekannt. Sofern sie nicht bekannt sind, können sie nach an sich bekannten Methoden hergestellt werden. So sind die Ausgangsstoffe der Formel II erhältlich durch Umsetzung von Propargylchloriden der Formel HC≡C-CR¹R²-Cl mit Hydroxypyridinen der Formel 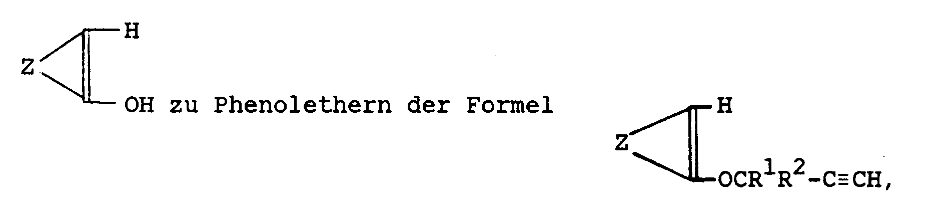
Cyclisierung zu Azachromenen entsprechend Formel I, worin R³ und R⁴ zusammen eine Bindung bedeuten, aber an Stelle des Restes R⁵ ein H-Atom steht, Anlagerung von HOBr zum Bromhydrin (IV) entsprechend Formel I, worin R³ OH bedeutet, aber Br an Stelle von R⁴ und H an Stelle von R⁵ steht, und Dehydrobromierung (Methode vgl. z. B. EP-A1-205292).

Zur Herstellung der Epoxide der Formel II kann man auch 2-Hydroxy-3-, -4-, -5- oder -6-aza-acetophenon mit einer Carbonylverbindung der Formel R¹-CO-R² zu 2-R¹-2-R²-5-, -6-, -7- oder -8-azachroman-4-on umsetzen, anschließend zu 2-R¹-2-R²-5-, -6-, -7- oder -8-azachroman-4-ol reduzieren, zu den entsprechenden 2-R¹-2-R²-5-, -6-, -7- oder -8-aza-2H-chromenen dehydratisieren und weiter zu 2-R¹-2-R²-5-, -6-, -7- oder -8-aza-3,4-epoxy-chroman umsetzen.

Als reaktionsfähige Derivate von III eignen sich die entsprechenden Salze, z. B. die Na- oder K-Salze, die auch in situ entstehen können.

Es ist zweckmäßig, in Gegenwart einer Base zu arbeiten. Als Basen eignen sich z. B. Alkalimetall- oder Erdalkalimetall-hydroxide, -hydride oder auch -amide wie NaOH, KOH, Ca(OH)₂, NaH, KH, CaH₂, NaNH₂, KNH₂, ferner organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II kann auch in situ hergestellt werden, z. B. durch Einwirkung einer Base auf das entsprechende Bromhydrin IV.

Die Verbindungen der Formel I können auch durch Reduktion entsprechender N-Oxide hergestellt werden, z. B. mit Hexachlordisilan in Chloroform bei Temperaturen zwischen -10 und +40°. Die N-Oxide sind z. B. erhältlich durch Reaktion der N-Oxide der Epoxide der Formel II mit Verbindungen der Formel III.

Eine Verbindung der Formel I, worin R³ = OH und R⁴ = H ist, kann durch Behandeln mit einem Dehydratisierungsmittel in eine Verbindung der Formel I, worin R³ und R⁴ zusammen eine Bindung bedeuten, umgewandelt werden. Das gelingt z. B. durch Einwirkung einer der angegebenen Basen, z. B. NaH, in einem der angegebenen Lösungsmittel, z. B. DMSO, bei Temperaturen zwischen 0 und 150 °.

Weiterhin kann man in einer Verbindung der Formel I einen oder mehrere der Reste R³ und/oder R⁵ in andere Reste R³ und/oder R⁵ umwandeln.

Beispielsweise ist es möglich, daß man ein H-Atom mittels einer Halogenierung durch ein Halogenatom oder einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder einen substituierten oder unsubstituierten 1H-2-Pyridon-1-ylrest (z. B. mit P₂S₅ oder mit Lawesson-Reagenz in Toluol) in den entsprechendne 1H-2-Thiopyridon-1-ylrest umwandelt.

Eine Nitrierung gelingt unter üblichen Bedingungen, z. B. mit einem Gemisch aus konzentrierter HNO₃ und konzentrierter H₂SO₄ bei Temperaturen zwischen 0 und 30 °. Die Nitrierung erfolgt überwiegend am Rest R⁵; andernfalls erhält man in der Regel Gemische, bei denen die Nitrogruppen am Rest R⁵ oder am Azachromanring stehen können.

Analoges gilt für die Halogenierung, die z. B. mit elementarem Chlor oder Brom in einem der üblichen inerten Lösungsmittel bei Temperaturen zwischen etwa 0 und 30 ° durchgeführt werden kann.

Eine OH-Gruppe kann durch Behandeln mit alkylierenden Mitteln in die entsprechende Alkoxygruppe umgewandelt werden. Als alkylierende Mittel eignen sich z. B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Die Alkylierung wird zweckmäßig in Gegenwart oder Abwesenheit eines der genannten inerten Lösungemittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z. B. Chloride oder Bromide) oder Anhydride von Carbonsäuren der Formel Ac-OH, z. B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z. B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z. B. Verbindungen der Formel I, worin R¹ = R², R³ = OH und R⁴ = H ist, zwei chirale Zentren; bei der Herstellung durch Reaktion von II mit III entsteht jedoch ganz überwiegend nur ein Racemat mit trans-Stellung der Substituenten R³ = OH und R⁵. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z. B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, R³ = OH) können ferner mit Hilfe chiraler Acylierungsreagenzien, z. B. D- oder L-α-Methylbenzylisocyanat, verestert und dann getrennt werden (vgl. EP-A1-120428). Die verschiedenen Formen der Diastereomeren können in an sich bekannter Weise, z. B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z. B. in Alkoholen wie Ethanol oder Isopropanol, Acetonitril, DMF, Dimethylacetamid, 1,2-Propandiol oder deren Gemischen untereinander oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und Geschmacks-und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z. B. Asthma, Inkontinenz der Harnblase.

Dabei werden die erfindungsgemäßen Substanzen in der Regel in Analogie zu bekannten Antianginosa bwz. Blutdrucksenkern, z. B. Nicorandil oder BRL-34915 [2,2-Dimethyl-4-(2-oxo-1-pyrrolidinyl)-6-cyan-chroman-3-ol; vgl. EP-A1-173848] verabreicht, vorzugsweise in Dosierungen zwischen etwa 0,1 und 50 mg, insbesondere zwischen 0,2 und 5 mg pro Dosierungseinheit. Die tägliche Dosierung liegt vorzugsweise zwischen etwa 0,001 und 1, insbesondere zwischen 0,003 und 0,1 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopezie einschließlich der androgenen Alopezie und der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Ein Gemisch von 1,77 g 2,2-Dimethyl-3,4-epoxy-6-aza-chroman ("IIa"; vgl. EP-A1-205292), 1,5 g 1H-2-Pyridon ("Pyridon"), 6 ml Ethanol und 0,6 ml Pyridin wird 2 Std. gekocht. Man dampft ein und chromatographiert den Rückstand über Kieselgel. Mit Ethylacetat/methanol (95:5) wird trans-2,2-Dimethyl-4-(1H-2-pyridon-1-yl)-6-aza-chroman-3-ol ("A") eluiert.
F. 208-210°.

Analog erhält man die trans-Epimeren folgender 6-Azachroman-3-ole:
2-Methyl-4-(1H-2-pyridon-1-yl)-
2,2-Dimethyl-4-(1H-2-thiopyridon-1-yl)
2,2-Dimethyl-4-(1H-6-methyl-2-pyridon-1-yl)-
2,2-Dimethyl-4-(1H-3-fluor-2-pyridon-1-yl)-
2,2-Dimethyl-4-(1H-3-chlor-2-pyridon-1-yl)-
2,2-Dimethyl-4-(1H-5-chlor-2-pyridon-1-yl)-
2,2-Dimethyl-4-(1H-6-chlor-2-pyridon-1-yl)-
2,2-Dimethyl-4-(1H-3-hydroxy-2-pyridon-1-yl)-
2,2-Dimethyl-4-(1H-4-hydroxy-2-pyridon-1-yl)-,
F. 208-210°
2,2-Dimethyl-4-(1H-5-hydroxy-2-pyridon-1-yl)-,
F. 207-208°
2,2-Dimethyl-4-(1H-3-methoxy-2-pyridon-1-yl)-
2,2-Dimethyl-4-(1H-5-methoxy-2-pyridon-1-yl)-
2,2-Dimethyl-4-(1H-3-acetoxy-2-pyridon-1-yl)-
2,2-Dimethyl-4-(1H-3-nitro-2-pyridon-1-yl)-
2,2-Dimethyl-4-(1H-5-nitro-2-pyridon-1-yl)-
2,2-Dimethyl-4-(1H-3-amino-2-pyridon-1-yl)-
2,2-Dimethyl-4-(1H-5-amino-2-pyridon-1-yl)-
2,2-Dimethyl-4-(1H-3-acetamido-2-pyridon-1-yl)-
2,2-Dimethyl-4-(1H-5-acetamido-2-pyridon-1-yl)-
2,2-Dimethyl-4-(1H-3-carboxy-2-pyridon-1-yl)-
2,2-Dimethyl-4-(1H-5-carboxy-2-pyridon-1-yl)-
2,2-Dimethyl-4-(1H-5-methoxycarbonyl-2-pyridon-1-yl)-
2,2-Dimethyl-4-(1H-3,5-dichlor-2-pyridon-1-yl)-
2,2-Dimethyl-4-(1H-3,5-dibrom-2-pyridon-1-yl)-
2,2-Dimethyl-4-(1H-3,5-dijod-2-pyridon-1-yl)-
2,2-Dimethyl-4-(1H-3-chlor-5-nitro-2-pyridon-1-yl)-
2,2-Dimethyl-4-(1H-3-nitro-5-chlor-2-pyridon-1-yl)-
2,2-Dimethyl-4-(1H-3-brom-5-nitro-2-pyridon-1-yl)-
2,2-Dimethyl-4-(1H-3-nitro-5-brom-2-pyridon-1-yl)-
2,2-Dimethyl-4-(1H-3,5-dinitro-2-pyridon-1-yl)-
2,2-Dimethyl-4-(1H-3-chlor-5-amino-2-pyridon-1-yl)-
2,2-Dimethyl-4-(1H-3-amino-5-chlor-2-pyridon-1-yl)-
2,2-Dimethyl-4-(1H-3-brom-5-amino-2-pyridon-1-yl)-
2,2-Dimethyl-4-(1H-3-amino-5-brom-2-pyridon-1-yl)-
2,2-Dimethyl-4-(1H-3-chlor-5-acetamido-2-pyridon-1-yl)-
2,2-Dimethyl-4-(1H-3-acetamido-5-chlor-2-pyridon-1-yl)-
2,2-Dimethyl-4-(1H-3-brom-5-acetamido-2-pyridon-1-yl)-
2,2-Dimethyl-4-(1H-acetamido-5-brom-2-pyridon-1-yl)-
2,2-Ethyl-2-methyl-4-(1H-2-pyridon-1-yl)-
2,2-Diethyl-4-(1H-2-pyridon-1-yl)-
2,2-Trimethylen-4-(1H-2-pyridon-1-yl)-
2,2-Tetramethylen-4-(1H-2-pyridon-1-yl)-
2,2-Pentamethylen-4-(1H-2-pyridon-1-yl)-
2,2-Hexamethylen-4-(1H-2-pyridon-1-yl)-
2,2-Dimethyl-4-(1H-2-pyridazinon-1-yl)-
2,2-Dimethyl-4-(4,5-dihydro-1H-6-pyridazinon-1-yl)-
2,2-Dimethyl-4-(1H-3-hydroxy-6-pyridazinon-1-yl)-,
F. 235-236°
2,2-Dimethyl-4-(1H-3-ethoxycarbonyl-6-pyridazinon-1-yl)-
2,2-Dimethyl-4-(1H-2-pyrimidinon-1-yl)-
2,2-Dimethyl-4-(1H-4-hydroxy-2-pyrimidinon-1-yl)-
2,2-Dimethyl-4-(1H-6-pyrimidinon-1-yl)-
2,2-Dimethyl-4-(1H-4-hydroxy-6-pyrimidinon-1-yl)-
2,2-Dimethyl-4-(1H-2-pyrazinon-1-yl)-, F. 268-272°.

### Beispiel 2

Analog Beispiel 1 erhält man aus 2,2-Dimethyl-3,4-epoxy-5-aza-chroman [erhältlich durch Kondensation von 2-Acetyl-3-hydroxypyridin mit Aceton zu 2,2-Dimethyl-5-aza-chroman-4-on (F. 102-103°), Reduktion mit NaBH₄ zu 2,2-Dimethyl-5-aza-chroman-4-ol (F. 65-67°), Dehydratisierung zu 2,2-Dimethyl-5-aza-2H-chromen und Epoxidierung] das trans-2,2-Dimethyl-4-(1H-2-pyridon-1-yl)-5-aza-chroman-3-ol, F. 244-246°.

Analog erhält man die trans-Epimeren folgender 5-Azachroman-3-ole:
2,2-Dimethyl-4-(1H-2-thiopyridon-1-yl)-
2,2-Dimethyl-4-(1H-3-chlor-2-pyridon-1-yl)-
2,2-Dimethyl-4-(1H-5-chlor-2-pyridon-1-yl)-
2,2-Dimethyl-4-(1H-6-chlor-2-pyridon-1-yl)-
2,2-Dimethyl-4-(1H-3-hydroxy-2-pyridon-1-yl)-
2,2-Dimethyl-4-(1H-4-hydroxy-2-pyridon-1-yl)-
2,2-Dimethyl-4-(1H-5-hydroxy-2-pyridon-1-yl)-
2,2-Dimethyl-4-(1H-3-methoxy-2-pyridon-1-yl)-
2,2-Dimethyl-4-(1H-3-acetoxy-2-pyridon-1-yl)-
2,2-Dimethyl-4-(1H-3-nitro-2-pyridon-1-yl)-
2,2-Dimethyl-4-(1H-5-nitro-2-pyridon-1-yl)-
2,2-Dimethyl-4-(1H-3-amino-2-pyridon-1-yl)-
2,2-Dimethyl-4-(1H-5-amino-2-pyridon-1-yl)-
2,2-Dimethyl-4-(1H-3-acetamido-2-pyridon-1-yl)-
2,2-Dimethyl-4-(1H-5-acetamido-2-pyridon-1-yl)-
2,2-Dimethyl-4-(1H-3-carboxy-2-pyridon-1-yl)-
2,2-Dimethyl-4-(1H-5-carboxy-2-pyridon-1-yl)-
2,2-Dimethyl-4-(1H-3,5-dichlor-2-pyridon-1-yl)-
2,2-Dimethyl-4-(1H-3,5-dibrom-2-pyridon-1-yl)-
2,2-Dimethyl-4-(1H-3-chlor-5-nitro-2-pyridon-1-yl)-
2,2-Dimethyl-4-(1H-3-nitro-5-chlor-2-pyridon-1-yl)-
2,2-Dimethyl-4-(1H-3-brom-5-nitro-2-pyridon-1-yl)-
2,2-Dimethyl-4-(1H-3-nitro-5-brom-2-pyridon-1-yl)-
2,2-Dimethyl-4-(1H-3,5-dinitro-2-pyridon-1-yl)-
2,2-Dimethyl-4-(1H-3-chlor-5-amino-2-pyridon-1-yl)-
2,2-Dimethyl-4-(1H-3-amino-5-chlor-2-pyridon-1-yl)-
2,2-Dimethyl-4-(1H-3-brom-5-amino-2-pyridon-1-yl)-
2,2-Dimethyl-4-(1H-3-amino-5-brom-2-pyridon-1-yl)-
2,2-Dimethyl-4-(1H-3-chlor-5-acetamido-2-pyridon-1-yl)-
2,2-Dimethyl-4-(1H-3-acetamido-5-chlor-2-pyridon-1-yl)-
2,2-Dimethyl-4-(1H-3-brom-5-acetamido-2-pyridon-1-yl)-
2,2-Dimethyl-4-(1H-3-acetamido-5-brom-2-pyridon-1-yl)-
2,2-Tetramethylen-4-(1H-2-pyridon-1-yl)-
2,2-Pentamethylen-4-(1H-2-pyridon-1-yl)-
2,2-Dimethyl-4-(1H-2-pyridazinon-1-yl)-
2,2-Dimethyl-4-(4,5-dihydro-1H-6-pyridazinon-1-yl)-
2,2-Dimethyl-4-(1H-3-hydroxy-6-pyridazinon-1-yl)-,
F. 132-133°
2,2-Dimethyl-4-(1H-3-ethoxycarbonyl-6-pyridazinon-1-yl)-
2,2-Dimethyl-4-(1H-2-pyrimidinon-1-yl)-
2,2-Dimethyl-4-(1H-4-hydroxy-2-pyrimidinon-1-yl)-
2,2-Dimethyl-4-(1H-6-pyrimidinon-1-yl)-
2,2-Dimethyl-4-(1H-4-hydroxy-6-pyrimidinon-1-yl)-
2,2-Dimethyl-4-(1H-2-pyrazinon-1-yl)-.

### Beispiel 3

Analog Beispiel 1 erhält man aus 2,2-Dimethyl-3,4-epoxy-7-aza-chroman [erhältlich durch Kondensation von 4-Acetyl-3-hydroxypyridin mit Aceton zu 2,2-Dimethyl-7-aza-chroman-4-on (F. 110-112°), Reduktion mit NaBH₄ zu 2,2-Dimethyl-7-aza-chroman-4-ol, Dehydratisierung zu 2,2-Dimethyl-7-aza-2H-chromen und Epoxidierung] das trans-2,2-Dimethyl-4-(1H-2-pyridon-1-yl)-7-aza-chroman-3-ol.

Analog erhält man die trans-Epimeren folgender 7-Azachroman-3-ole:
2,2-Dimethyl-4-(1H-2-thiopyridon-1-yl)-
2,2-Dimethyl-4-(1H-3-chlor-2-pyridon-1-yl)-
2,2-Dimethyl-4-(1H-5-chlor-2-pyridon-1-yl)-
2,2-Dimethyl-4-(1H-6-chlor-2-pyridon-1-yl)-
2,2-Dimethyl-4-(1H-3-hydroxy-2-pyridon-1-yl)-
2,2-Dimethyl-4-(1H-4-hydroxy-2-pyridon-1-yl)-
2,2-Dimethyl-4-(1H-5-hydroxy-2-pyridon-1-yl)-
2,2-Dimethyl-4-(1H-3-methoxy-2-pyridon-1-yl)-
2,2-Dimethyl-4-(1H-3-acetoxy-2-pyridon-1-yl)-
2,2-Dimethyl-4-(1H-3-nitro-2-pyridon-1-yl)-
2,2-Dimethyl-4-(1H-5-nitro-2-pyridon-1-yl)-
2,2-Dimethyl-4-(1H-3-amino-2-pyridon-1-yl)-
2,2-Dimethyl-4-(1H-5-amino-2-pyridon-1-yl)-
2,2-Dimethyl-4-(1H-3-acetamido-2-pyridon-1-yl)-
2,2-Dimethyl-4-(1H-5-acetamido-2-pyridon-1-yl)-
2,2-Dimethyl-4-(1H-3-carboxy-2-pyridon-1-yl)-
2,2-Dimethyl-4-(1H-5-carboxy-2-pyridon-1-yl)-
2,2-Dimethyl-4-(1H-3,5-dichlor-2-pyridon-1-yl)-
2,2-Dimethyl-4-(1H-3,5-dibrom-2-pyridon-1-yl)-
2,2-Dimethyl-4-(1H-3-chlor-5-nitro-2-pyridon-1-yl)-
2,2-Dimethyl-4-(1H-3-nitro-5-chlor-2-pyridon-1-yl)-
2,2-Dimethyl-4-(1H-3-brom-5-nitro-2-pyridon-1-yl)-
2,2-Dimethyl-4-(1H-3-nitro-5-brom-2-pyridon-1-yl)-
2,2-Dimethyl-4-(1H-3,5-dinitro-2-pyridon-1-yl)-
2,2-Dimethyl-4-(1H-3-chlor-5-amino-2-pyridon-1-yl)-
2,2-Dimethyl-4-(1H-3-amino-5-chlor-2-pyridon-1-yl)-
2,2-Dimethyl-4-(1H-3-brom-5-amino-2-pyridon-1-yl)-
2,2-Dimethyl-4-(1H-3-amino-5-brom-2-pyridon-1-yl)-
2,2-Dimethyl-4-(1H-3-chlor-5-acetamido-2-pyridon-1-yl)-
2,2-Dimethyl-4-(1H-3-acetamido-5-chlor-2-pyridon-1-yl)-
2,2-Dimethyl-4-(1H-3-brom-5-acetamido-2-pyridon-1-yl)-
2,2-Dimethyl-4-(1H-3-acetamido-5-brom-2-pyridon-1-yl)-
2,2-Tetramethylen-4-(1H-2-pyridon-1-yl)-
2,2-Pentamethylen-4-(1H-2-pyridon-1-yl)-
2,2-Dimethyl-4-(1H-2-pyridazinon-1-yl)-
2,2-Dimethyl-4-(4,5-dihydro-1H-6-pyridazinon-1-yl)-
2,2-Dimethyl-4-(1H-3-hydroxy-6-pyridazinon-1-yl)-
2,2-Dimethyl-4-(1H-3-ethoxycarbonyl-6-pyridazinon-1-yl)-
2,2-Dimethyl-4-(1H-2-pyrimidinon-1-yl)-
2,2-Dimethyl-4-(1H-4-hydroxy-2-pyrimidinon-1-yl)-
2,2-Dimethyl-4-(1H-6-pyrimidinon-1-yl)-
2,2-Dimethyl-4-(1H-4-hydroxy-6-pyrimidinon-1-yl)-
2,2-Dimethyl-4-(1H-2-pyrazinon-1-yl)-.

### Beispiel 4

Analog Beispiel 1 erhält man aus 2,2-Dimethyl-3,4-epoxy-8-aza-chroman [erhältlich durch Kondensation von 3-Acetyl-2-hydroxypyridin mit Aceton zu 2,2-Dimethyl-8-aza-chroman-4-on, Reduktion mit NaBH₄ zu 2,2-Dimethyl-8-aza-chroman-4-ol, Dehydratisierung zu 2,2-Dimethyl-8-aza-2H-chromen und Epoxidierung] das trans-2,2-Dimethyl-4-(1H-2-pyridon-1-yl)-8-aza-chroman-3-ol, F. 179-180°.

Analog erhält man die trans-Epimeren folgender 8-Azachroman-3-ole:
2,2-Dimethyl-4-(1H-2-thiopyridon-1-yl)-
2,2-Dimethyl-4-(1H-3-chlor-2-pyridon-1-yl)-
2,2-Dimethyl-4-(1H-5-chlor-2-pyridon-1-yl)-
2,2-Dimethyl-4-(1H-6-chlor-2-pyridon-1-yl)-
2,2-Dimethyl-4-(1H-3-hydroxy-2-pyridon-1-yl)-
2,2-Dimethyl-4-(1H-4-hydroxy-2-pyridon-1-yl)-
2,2-Dimethyl-4-(1H-5-hydroxy-2-pyridon-1-yl)-
2,2-Dimethyl-4-(1H-3-methoxy-2-pyridon-1-yl)-
2,2-Dimethyl-4-(1H-3-acetoxy-2-pyridon-1-yl)-
2,2-Dimethyl-4-(1H-3-nitro-2-pyridon-1-yl)-
2,2-Dimethyl-4-(1H-5-nitro-2-pyridon-1-yl)-
2,2-Dimethyl-4-(1H-3-amino-2-pyridon-1-yl)-
2,2-Dimethyl-4-(1H-5-amino-2-pyridon-1-yl)-
2,2-Dimethyl-4-(1H-3-acetamido-2-pyridon-1-yl)-
2,2-Dimethyl-4-(1H-5-acetamido-2-pyridon-1-yl)-
2,2-Dimethyl-4-(1H-3-carboxy-2-pyridon-1-yl)-
2,2-Dimethyl-4-(1H-5-carboxy-2-pyridon-1-yl)-
2,2-Dimethyl-4-(1H-3,5-dichlor-2-pyridon-1-yl)-
2,2-Dimethyl-4-(1H-3,5-dibrom-2-pyridon-1-yl)-
2,2-Dimethyl-4-(1H-3-chlor-5-nitro-2-pyridon-1-yl)-
2,2-Dimethyl-4-(1H-3-nitro-5-chlor-2-pyridon-1-yl)-
2,2-Dimethyl-4-(1H-3-brom-5-nitro-2-pyridon-1-yl)-
2,2-Dimethyl-4-(1H-3-nitro-5-brom-2-pyridon-1-yl)-
2,2-Dimethyl-4-(1H-3,5-dinitro-2-pyridon-1-yl)-
2,2-Dimethyl-4-(1H-3-chlor-5-amino-2-pyridon-1-yl)-
2,2-Dimethyl-4-(1H-3-amino-5-chlor-2-pyridon-1-yl)-
2,2-Dimethyl-4-(1H-3-brom-5-amino-2-pyridon-1-yl)-
2,2-Dimethyl-4-(1H-3-amino-5-brom-2-pyridon-1-yl)-
2,2-Dimethyl-4-(1H-3-chlor-5-acetamido-2-pyridon-1-yl)-
2,2-Dimethyl-4-(1H-3-acetamido-5-chlor-2-pyridon-1-yl)-
2,2-Dimethyl-4-(1H-3-brom-5-acetamido-2-pyridon-1-yl)-
2,2-Dimethyl-4-(1H-3-acetamido-5-brom-2-pyridon-1-yl)-
2,2-Tetramethylen-4-(1H-2-pyridon-1-yl)-
2,2-Pentamethylen-4-(1H-2-pyridon-1-yl)-
2,2-Dimethyl-4-(1H-2-pyridazinon-1-yl)-
2,2-Dimethyl-4-(4,5-dihydro-1H-6-pyridazinon-1-yl)-
2,2-Dimethyl-4-(1H-3-hydroxy-6-pyridazinon-1-yl)-
2,2-Dimethyl-4-(1H-3-ethoxycarbonyl-6-pyridazinon-1-yl)-
2,2-Dimethyl-4-(1H-2-pyrimidinon-1-yl)-
2,2-Dimethyl-4-(1H-4-hydroxy-2-pyrimidinon-1-yl)-
2,2-Dimethyl-4-(1H-6-pyrimidinon-1-yl)-
2,2-Dimethyl-4-(1H-4-hydroxy-6-pyrimidinon-1-yl)-
2,2-Dimethyl-4-(1H-2-pyrazinon-1-yl)-.

### Beispiel 5

In eine Lösung von 2,1 g "A" in 105 ml DMF werden unter N₂ 463 mg 80%iges NaH (in Paraffinöl) eingetragen. Man rührt 2 Std. bei 70°, zersetzt bei 5-10° tropfenweise mit Wasser, arbeitet wie üblich auf und erhält 2,2-Dimethyl-4-(1H-2-pyridon-1-yl)-6-aza-2H-chromen ("B"). F. 118-120°.

Analog erhält man durch Dehydratisierung der entsprechenden 5-, 6-, 7- oder 8-Azachroman-3-ole:
2,2-Dimethyl-4-(1H-2-pyridon-1-yl)-5-aza-2H-chromen,
F. 147-148°
2,2-Dimethyl-4-(1H-2-pyridon-1-yl)-7-aza-2H-chromen
2,2-Dimethyl-4-(1H-2-pyridon-1-yl)-8-aza-2H-chromen
2,2-Dimethyl-4-(1H-2-thiopyridon-1-yl)-5-aza-2H-chromen
2,2-Dimethyl-4-(1H-2-thiopyridon-1-yl)-6-aza-2H-chromen
2,2-Dimethyl-4-(1H-2-thiopyridon-1-yl)-7-aza-2H-chromen
2,2-Dimethyl-4-(1H-2-thiopyridon-1-yl)-8-aza-2H-chromen
2,2-Dimethyl-4-(1H-3-chlor-2-pyridon-1-yl)-5-aza-2H-chromen
2,2-Dimethyl-4-(1H-3-chlor-2-pyridon-1-yl)-6-aza-2H-chromen
2,2-Dimethyl-4-(1H-3-chlor-2-pyridon-1-yl)-7-aza-2H-chromen
2,2-Dimethyl-4-(1H-3-chlor-2-pyridon-1-yl)-8-aza-2H-chromen
2,2-Dimethyl-4-(1H-5-chlor-2-pyridon-1-yl)-5-aza-2H-chromen
2,2-Dimethyl-4-(1H-5-chlor-2-pyridon-1-yl)-6-aza-2H-chromen
2,2-Dimethyl-4-(1H-5-chlor-2-pyridon-1-yl)-7-aza-2H-chromen
2,2-Dimethyl-4-(1H-5-chlor-2-pyridon-1-yl)-8-aza-2H-chromen
2,2-Dimethyl-4-(1H-6-chlor-2-pyridon-1-yl)-5-aza-2H-chromen
2,2-Dimethyl-4-(1H-6-chlor-2-pyridon-1-yl)-6-aza-2H-chromen
2,2-Dimethyl-4-(1H-6-chlor-2-pyridon-1-yl)-7-aza-2H-chromen
2,2-Dimethyl-4-(1H-6-chlor-2-pyridon-1-yl)-8-aza-2H-chromen
2,2-Dimethyl-4-(1H-3-hydroxy-2-pyridon-1-yl)-5-aza-2H-chromen
2,2-Dimethyl-4-(1H-3-hydroxy-2-pyridon-1-yl)-6-aza-2H-chromen
2,2-Dimethyl-4-(1H-3-hydroxy-2-pyridon-1-yl)-7-aza-2H-chromen
2,2-Dimethyl-4-(1H-3-hydrxoy-2-pyridon-1-yl)-8-aza-2H-chromen
2,2-Dimethyl-4-(1H-4-hydroxy-2-pyridon-1-yl)-5-aza-2H-chromen
2,2-Dimethyl-4-(1H-4-hydroxy-2-pyridon-1-yl)-6-aza-2H-chromen
2,2-Dimethyl-4-(1H-4-hydroxy-2-pyridon-1-yl)-7-aza-2H-chromen
2,2-Dimethyl-4-(1H-4-hydrxoy-2-pyridon-1-yl)-8-aza-2H-chromen
2,2-Dimethyl-4-(1H-5-hydroxy-2-pyridon-1-yl)-5-aza-2H-chromen
2,2-Dimethyl-4-(1H-5-hydroxy-2-pyridon-1-yl)-6-aza-2H-chromen
2,2-Dimethyl-4-(1H-5-hydroxy-2-pyridon-1-yl)-7-aza-2H-chromen
2,2-Dimethyl-4-(1H-5-hydrxoy-2-pyridon-1-yl)-8-aza-2H-chromen
2,2-Dimethyl-4-(1H-3-methoxy-2-pyridon-1-yl)-5-aza-2H-chromen
2,2-Dimethyl-4-(1H-3-methoxy-2-pyridon-1-yl)-6-aza-2H-chromen
2,2-Dimethyl-4-(1H-3-methoxy-2-pyridon-1-yl)-7-aza-2H-chromen
2,2-Dimethyl-4-(1H-3-methoxy-2-pyridon-1-yl)-8-aza-2H-chromen
2,2-Dimethyl-4-(1H-3-acetxy-2-pyridon-1-yl)-5-aza-2H-chromen
2,2-Dimethyl-4-(1H-3-acetoxy-2-pyridon-1-yl)-6-aza-2H-chromen
2,2-Dimethyl-4-(1H-3-acetoxy-2-pyridon-1-yl)-7-aza-2H-chromen
2,2-Dimethyl-4-(1H-3-acetoxy-2-pyridon-1-yl)-8-aza-2H-chromen
2,2-Dimethyl-4-(1H-3-nitro-2-pyridon-1-yl)-5-aza-2H-chromen
2,2-Dimethyl-4-(1H-3-nitro-2-pyridon-1-yl)-6-aza-2H-chromen
2,2-Dimethyl-4-(1H-3-nitro-2-pyridon-1-yl)-7-aza-2H-chromen
2,2-Dimethyl-4-(1H-3-nitro-2-pyridon-1-yl)-8-aza-2H-chromen
2,2-Dimethyl-4-(1H-5-nitro-2-pyridon-1-yl)-5-aza-2H-chromen
2,2-Dimethyl-4-(1H-5-nitro-2-pyridon-1-yl)-6-aza-2H-chromen
2,2-Dimethyl-4-(1H-5-nitro-2-pyridon-1-yl)-7-aza-2H-chromen
2,2-Dimethyl-4-(1H-5-nitro-2-pyridon-1-yl)-8-aza-2H-chromen
2,2-Dimethyl-4-(1H-3-amino-2-pyridon-1-yl)-5-aza-2H-chromen
2,2-Dimethyl-4-(1H-3-amino-2-pyridon-1-yl)-6-aza-2H-chromen
2,2-Dimethyl-4-(1H-3-amino-2-pyridon-1-yl)-7-aza-2H-chromen
2,2-Dimethyl-4-(1H-3-amino-2-pyridon-1-yl)-8-aza-2H-chromen
2,2-Dimethyl-4-(1H-5-amino-2-pyridon-1-yl)-5-aza-2H-chromen
2,2-Dimethyl-4-(1H-5-amino-2-pyridon-1-yl)-6-aza-2H-chromen
2,2-Dimethyl-4-(1H-5-amino-2-pyridon-1-yl)-7-aza-2H-chromen
2,2-Dimethyl-4-(1H-5-amino-2-pyridon-1-yl)-8-aza-2H-chromen
2,2-Dimethyl-4-(1H-3-acetamido-2-pyridon-1-yl)-5-aza-2H-chromen
2,2-Dimethyl-4-(1H-3-acetamido-2-pyridon-1-yl)-6-aza-2H-chromen
2,2-Dimethyl-4-(1H-3-acetamido-2-pyridon-1-yl)-7-aza-2H-chromen
2,2-Dimethyl-4-(1H-3-acetamido-2-pyridon-1-yl)-8-aza-2H-chromen
2,2-Dimethyl-4-(1H-5-acetamido-2-pyridon-1-yl)-5-aza-2H-chromen
2,2-Dimethyl-4-(1H-5-acetamdio-2-pyridon-1-yl)-6-aza-2H-chromen
2,2-Dimethyl-4-(1H-5-acetamido-2-pyridon-1-yl)-7-aza-2H-chromen
2,2-Dimethyl-4-(1H-5-acetamido-2-pyridon-1-yl)-8-aza-2H-chromen
2,2-Dimethyl-4-(1H-3-carboxy-2-pyridon-1-yl)-5-aza-2H-chromen
2,2-Dimethyl-4-(1H-3-carboxy-2-pyridon-1-yl)-6-aza-2H-chromen
2,2-Dimethyl-4-(1H-3-carboxy-2-pyridon-1-yl)-7-aza-2H-chromen
2,2-Dimethyl-4-(1H-3-carboxy-2-pyridon-1-yl)-8-aza-2H-chromen
2,2-Dimethyl-4-(1H-5-carboxy-2-pyridon-1-yl)-5-aza-2H-chromen
2,2-Dimethyl-4-(1H-5-carboxy-2-pyridon-1-yl)-6-aza-2H-chromen
2,2-Dimethyl-4-(1H-5-carboxy-2-pyridon-1-yl)-7-aza-2H-chromen
2,2-Dimethyl-4-(1H-5-carboxy-2-pyridon-1-yl)-8-aza-2H-chromen
2,2-Dimethyl-4-(1H-3,5-dichlor-2-pyridon-1-yl)-5-aza-2H-chromen
2,2-Dimethyl-4-(1H-3,5-dichlor-2-pyridon-1-yl)-6-aza-2H-chromen
2,2-Dimethyl-4-(1H-3,5-dichlor-2-pyridon-1-yl)-7-aza-2H-chromen
2,2-Dimethyl-4-(1H-3,5-dichlor-2-pyridon-1-yl)-8-aza-2H-chromen
2,2-Dimethyl-4-(1H-3,5-dibrom-2-pyridon-1-yl)-5-aza-2H-chromen
2,2-Dimethyl-4-(1H-3,5-dibrom-2-pyridon-1-yl)-6-aza-2H-chromen
2,2-Dimethyl-4-(1H-3,5-dibrom-2-pyridon-1-yl)-7-aza-2H-chromen
2,2-Dimethyl-4-(1H-3,5-dibrom-2-pyridon-1-yl)-8-aza-2H-chromen
2,2-Dimethyl-4-(1H-3-chlor-5-nitro-2-pyridon-1-yl)-5-aza-2H-chromen
2,2-Dimethyl-4-(1H-3-chlor-5-nitro-2-pyridon-1-yl)-6-aza-2H-chromen
2,2-Dimethyl-4-(1H-3-chlor-5-nitro-2-pyridon-1-yl)-7-aza-2H-chromen
2,2-Dimethyl-4-(1H-3-chlor-5-nitro-2-pyridon-1-yl)-8-aza-2H-chromen
2,2-Dimethyl-4-(1H-3-nitro-5-chlor-2-pyridon-1-yl)-5-aza-2H-chromen
2,2-Dimethyl-4-(1H-3-nitro-5-chlor-2-pyridon-1-yl)-6-aza-2H-chromen
2,2-Dimethyl-4-(1H-3-nitro-5-chlor-2-pyridon-1-yl)-7-aza-2H-chromen
2,2-Dimethyl-4-(1H-3-nitro-5-chlor-2-pyridon-1-yl)-8-aza-2H-chromen
2,2-Dimethyl-4-(1H-3-brom-5-nitro-2-pyridon-1-yl)-5-aza-2H-chromen
2,2-Dimethyl-4-(1H-3-brom-5-nitro-2-pyridon-1-yl)-6-aza-2H-chromen
2,2-Dimethyl-4-(1H-3-brom-5-nitro-2-pyridon-1-yl)-7-aza-2H-chromen
2,2-Dimethyl-4-(1H-3-brom-5-nitro-2-pyridon-1-yl)-8-aza-2H-chromen
2,2-Dimethyl-4-(1H-3-nitro-5-brom-2-pyridon-1-yl)-5-aza-2H-chromen
2,2-Dimethyl-4-(1H-3-nitro-5-brom-2-pyridon-1-yl)-6-aza-2H-chromen
2,2-Dimethyl-4-(1H-3-nitro-5-brom-2-pyridon-1-yl)-7-aza-2H-chromen
2,2-Dimethyl-4-(1H-3-nitro-5-brom-2-pyridon-1-yl)-8-aza-2H-chromen
2,2-Dimethyl-4-(1H-3,5-dinitro-2-pyridon-1-yl)-5-aza-2H-chromen
2,2-Dimethyl-4-(1H-3,5-dinitro-2-pyridon-1-yl)-6-aza-2H-chromen
2,2-Dimethyl-4-(1H-3,5-dinitro-2-pyridon-1-yl)-7-aza-2H-chromen
2,2-Dimethyl-4-(1H-3,5-dinitro-2-pyridon-1-yl)-8-aza-2H-chromen
2,2-Dimethyl-4-(1H-3-chlor-5-amino-2-pyridon-1-yl)-5-aza-2H-chromen
2,2-Dimethyl-4-(1H-3-chlor-5-amino-2-pyridon-1-yl)-6-aza-2H-chromen
2,2-Dimethyl-4-(1H-3-chlor-5-amino-2-pyridon-1-yl)-7-aza-2H-chromen
2,2-Dimethyl-4-(1H-3-chlor-5-amino-2-pyridon-1-yl)-8-aza-2H-chromen
2,2-Dimethyl-4-(1H-3-amino-5-chlor-2-pyridon-1-yl)-5-aza-2H-chromen
2,2-Dimethyl-4-(1H-3-amino-5-chlor-2-pyridon-1-yl)-6-aza-2H-chromen
2,2-Dimethyl-4-(1H-3-amino-5-chlor-2-pyridon-1-yl)-7-aza-2H-chromen
2,2-Dimethyl-4-(1H-3-amino-5-chlor-2-pyridon-1-yl)-8-aza-2H-chromen
2,2-Dimethyl-4-(1H-3-brom-5-amino-2-pyridon-1-yl)-5-aza-2H-chromen
2,2-Dimethyl-4-(1H-3-brom-5-amino-2-pyridon-1-yl)-6-aza-2H-chromen
2,2-Dimethyl-4-(1H-3-brom-5-amino-2-pyridon-1-yl)-7-aza-2H-chromen
2,2-Dimethyl-4-(1H-3-brom-5-amino-2-pyridon-1-yl)-8-aza-2H-chromen
2,2-Dimethyl-4-(1H-3-amino-5-brom-2-pyridon-1-yl)-5-aza-2H-chromen
2,2-Dimethyl-4-(1H-3-amino-5-brom-2-pyridon-1-yl)-6-aza-2H-chromen
2,2-Dimethyl-4-(1H-3-amino-5-brom-2-pyridon-1-yl)-7-aza-2H-chromen
2,2-Dimethyl-4-(1H-3-amino-5-brom-2-pyridon-1-yl)-8-aza-2H-chromen
2,2-Dimethyl-4-(1H-3-chlor-5-acetamido-2-pyridon-1-yl)-5-aza-2H-chromen
2,2-Dimethyl-4-(1H-3-chlor-5-acetamido-2-pyridon-1-yl)-6-aza-2H-chromen
2,2-Dimethyl-4-(1H-3-chlor-5-acetamido-2-pyridon-1-yl)-7-aza-2H-chromen
2,2-Dimethyl-4-(1H-3-chlor-5-acetamido-2-pyridon-1-yl)-8-aza-2H-chromen
2,2-Dimethyl-4-(1H-3-acetamido-5-chlor-2-pyridon-1-yl)-5-aza-2H-chromen
2,2-Dimethyl-4-(1H-3-acetamido-5-chlor-2-pyridon-1-yl)-6-aza-2H-chromen
2,2-Dimethyl-4-(1H-3-acetamido-5-chlor-2-pyridon-1-yl)-7-aza-2H-chromen
2,2-Dimethyl-4-(1H-3-acetamido-5-chlor-2-pyridon-1-yl)-8-aza-2H-chromen
2,2-Dimethyl-4-(1H-3-brom-5-acetamido-2-pyridon-1-yl)-5-aza-2H-chromen
2,2-Dimethyl-4-(1H-3-brom-5-acetamido-2-pyridon-1-yl)-6-aza-2H-chromen
2,2-Dimethyl-4-(1H-3-brom-5-acetamido-2-pyridon-1-yl)-7-aza-2H-chromen
2,2-Dimethyl-4-(1H-3-brom-5-acetamido-2-pyridon-1-yl)-8-aza-2H-chromen
2,2-Dimethyl-4-(1H-3-acetamido-5-brom-2-pyridon-1-yl)-5-aza-2H-chromen
2,2-Dimethyl-4-(1H-3-acetamido-5-brom-2-pyridon-1-yl)-6-aza-2H-chromen
2,2-Dimethyl-4-(1H-3-acetamido-5-brom-2-pyridon-1-yl)-7-aza-2H-chromen
2,2-Dimethyl-4-(1H-3-acetamido-5-brom-2-pyridon-1-yl)-8-aza-2H-chromen
2,2-Tetramethylen-4-(1H-2-pyridon-1-yl)-5-aza-2H-chromen
2,2-Tetramethylen-4-(1H-2-pyridon-1-yl)-6-aza-2H-chromen
2,2-Tetramethylen-4-(1H-2-pyridon-1-yl)-7-aza-2H-chromen
2,2-Tetramethylen-4-(1H-2-pyridon-1-yl)-8-aza-2H-chromen
2,2-Pentamethylen-4-(1H-2-pyridon-1-yl)-5-aza-2H-chromen
2,2-Pentamethylen-4-(1H-2-pyridon-1-yl)-6-aza-2H-chromen
2,2-Pentamethylen-4-(1H-2-pyridon-1-yl)-7-aza-2H-chromen
2,2-Pentamethylen-4-(1H-2-pyridon-1-yl)-8-aza-2H-chromen
2,2-Dimethyl-4-(1H-2-pyridazinon-1-yl)-5-aza-2H-chromen
2,2-Dimethyl-4-(1H-2-pyridazinon-1-yl)-6-aza-2H-chromen
2,2-Dimethyl-4-(1H-2-pyridazinon-1-yl)-7-aza-2H-chromen
2,2-Dimethyl-4-(1H-2-pyridazinon-1-yl)-8-aza-2H-chromen
2,2-Dimethyl-4-(4,5-dihydro-1H-6-pyridazinon-1-yl)-5-aza-2H-chromen
2,2-Dimethyl-4-(4,5-dihydro-1H-6-pyridazinon-1-yl)-6-aza-2H-chromen
2,2-Dimethyl-4-(4,5-dihydro-1H-6-pyridazinon-1-yl)-7-aza-2H-chromen
2,2-Dimethyl-4-(4,5-dihydro-1H-6-pyridazinon-1-yl)-8-aza-2H-chromen
2,2-Dimethyl-4-(1H-3-hydroxy-6-pyridazinon-1-yl)-5-aza-2H-chromen
2,2-Dimethyl-4-(1H-3-hydroxy-6-pyridazinon-1-yl)-6-aza-2H-chromen
2,2-Dimethyl-4-(1H-3-hydroxy-6-pyridazinon-1-yl)-7-aza-2H-chromen
2,2-Dimethyl-4-(1H-3-hydroxy-6-pyridazinon-1-yl)-8-aza-2H-chromen
2,2-Dimethyl-4-(1H-3-ethoxycarbonyl-6-pyridazinon-1-yl)-5-aza-2H-chromen
2,2-Dimethyl-4-(1H-3-ethoxycarbonyl-6-pyridazinon-1-yl)-6-aza-2H-chromen
2,2-Dimethyl-4-(1H-3-ethoxycarbonyl-6-pyridazinon-1-yl)-7-aza-2H-chromen
2,2-Dimethyl-4-(1H-3-ethoxycarbonyl-6-pyridazinon-1-yl)-8-aza-2H-chromen
2,2-Dimethyl-4-(1H-2-pyrimidinon-1-yl)-5-aza-2H-chromen
2,2-Dimethyl-4-(1H-2-pyrimidinon-1-yl)-6-aza-2H-chromen
2,2-Dimethyl-4-(1H-2-pyrimidinon-1-yl)-7-aza-2H-chromen
2,2-Dimethyl-4-(1H-2-pyrimidinon-1-yl)-8-aza-2H-chromen
2,2-Dimethyl-4-(1H-4-hydroxy-2-pyrimidinon-1-yl)-5-aza-2H-chromen
2,2-Dimethyl-4-(1H-4-hydroxy-2-pyrimidinon-1-yl)-6-aza-2H-chromen
2,2-Dimethyl-4-(1H-4-hydroxy-2-pyrimidinon-1-yl)-7-aza-2H-chromen
2,2-Dimethyl-4-(1H-4-hydroxy-2-pyrimidinon-1-yl)-8-aza-2H-chromen
2,2-Dimethyl-4-(1H-6-pyrimidinon-1-yl)-5-aza-2H-chromen
2,2-Dimethyl-4-(1H-6-pyrimidinon-1-yl)-6-aza-2H-chromen
2,2-Dimethyl-4-(1H-6-pyrimidinon-1-yl)-7-aza-2H-chromen
2,2-Dimethyl-4-(1H-6-pyrimidinon-1-yl)-8-aza-2H-chromen
2,2-Dimethyl-4-(1H-6-hydroxy-2-pyrimidinon-1-yl)-5-aza-2H-chromen
2,2-Dimethyl-4-(1H-6-hydroxy-2-pyrimidinon-1-yl)-6-aza-2H-chromen
2,2-Dimethyl-4-(1H-6-hydroxy-2-pyrimidinon-1-yl)-7-aza-2H-chromen
2,2-Dimethyl-4-(1H-6-hydroxy-2-pyrimidinon-1-yl)-8-aza-2H-chromen
2,2-Dimethyl-4-(1H-2-pyrazinon-1-yl)-5-aza-2H-chromen
2,2-Dimethyl-4-(1H-2-pyrazinon-1-yl)-6-aza-2H-chromen
2,2-Dimethyl-4-(1H-2-pyrazinon-1-yl)-7-aza-2H-chromen
2,2-Dimethyl-4-(1H-2-pyrazinon-1-yl)-8-aza-2H-chromen.

### Beispiel 6

Unter Rühren werden 0,4 g einer 60 %igen Öldispersion von NaH zu einer Lösung von 2,82 g trans-2,2-Dimethyl-3-brom-6-aza-chroman-4-ol in 15 ml DMSO gegeben. Man rührt 1 Std., wobei intermediär 2,2-Dimethyl-3,4-epoxy-6-aza-chroman entsteht. Man gibt 1,43 g 1H-2-Pyridon und weitere 0,5 g NaH-Dispersion hinzu und rührt 16 Std. bei 20 °. Nach Aufarbeitung analog Beispiel 1 erhält man "A", F. 208-210°.

### Beispiel 7

Ein Gemisch von 2 g "A", 11,7 ml Ameisensäure und 3,3 ml Acetanhydrid wird 16 Std. bei 20° stehengelassen und anschließend 2 Std. auf 40-42° erwärmt. Nach Eindampfen und üblicher Aufarbeitung erhält man 2,2-Dimethyl-3-formyloxy-4-(1H-2-pyridon-1-yl)-6-aza-chroman.

### Beispiel 8

Ein Gemisch von 1 g "A" und 5 ml Acetanhydrid wird 1 Std. gekocht. Man kühlt ab, arbeitet wie üblich auf und erhält 2,2-Dimethyl-3-acetoxy-4-(1H-2-pyridon-1-yl)-6-aza-chroman.

### Beispiel 9

Man suspendiert 2,96 g "A" in 100 ml Wasser und tropft unter Rühren bei 10-20 ° 3,2 g Brom hinzu. Die Substanz löst sich, 2,2-Dimethyl-4-(1H-3,5-dibrom-2-pyridon-1-yl)-6-aza-chroman-3-ol fällt aus und wird abfiltriert.

### Beispiel 10

Man löst 2,78 g "B" in einem Gemisch von 10 ml konzentrierter Salpetersäure (68 %ig; D. 1,41) und 12 ml konzentrierter Schwefelsäure, rührt 3 Std. bei 20 °, gießt auf Eis, filtriert, wäscht mit Wasser und erhält ein Gemisch von 2,2-Dimethyl-4-(1H-3- und -5-nitro-pyridon-1-yl)-6-aza-2H-chromen, das chromatographisch getrennt werden kann.

### Beispiel 11

Eine Lösung von 1 g 2,2-Dimethyl-4-(1H-3-nitro-2-pyridon-1-yl)-6-aza-chroman-3-ol in 25 ml Methanol wird bei 20° und 1 bar an 0,5 g 5 %igem Pd-C bis zum Stillstand hydriert. Man filtriert, dampft ein und erhält 2,2-Dimethyl-4-(1H-3-amino-2-pyridon-1-yl)-6-aza-chroman-3-ol.

### Beispiel 12

Eine Lösung von 1 g 2,2-Dimethyl-4-(1H-3-amino-2-pyridon-1-yl)-6-aza-2H-chromen in 15 ml HCOOH und 1 ml Pyridin wird 19 Std. gekocht und eingedampft. Nach üblicher Aufarbeitung erhält man 2,2-Dimethyl-4-(1H-3-formamido-2-pyridon-1-yl)-6-aza-2H-chromen.

### Beispiel 13

Ein Gemisch von 1 g 2,2-Dimethyl-4-(1H-5-amino-2-pyridon-1-yl)-6-aza-2H-chromen, 10 ml Acetanhydrid und 10 ml Pyridin wird 16 Std. bei 20 ° stehengelassen. Man dampft ein, reinigt chromatographisch und erhält 2,2-Dimethyl-4-(1H-5-acetamido-2-pyridon-1-yl)-6-aza-2H-chromen.

### Beispiel 14

Ein Gemisch von 272 mg "A", 808 mg Lawesson-Reagenz und 50 ml Toluol wird 1 Std. unter N₂ gekocht. Übliche Aufarbeitung gibt 2,2-Dimethyl-4-(1H-2-thiopyridon-1-yl)-6-aza-chroman-3-ol.

Analog erhält man aus "B" das 2,2-Dimethyl-4-(1H-2-thiopyridon-1-yl)-6-aza-2H-chromen.

### Beispiel 15

Zu einer Suspension von 500 mg 2,2-Dimethyl-4-(1H-2-pyridon-1-yl)-7-aza-chroman-3-ol-7-N-oxid (erhältlich aus 2,2-Dimethyl-3,4-epoxy-7-aza-chroman-7-N-oxid und Pyridon) in 120 ml CHCl₃ tropft man unter Rühren und N₂-Atmosphäre bei 10-15° eine Lösung von 4,66 g Hexachlordisilan in 30 ml CHCl₃, rührt noch 1 Std. bei 20°, tropft bei 0-5° 36,4 ml 10%ige Natronlauge hinzu, arbeitet wie üblich auf und erhält "A", F. 208-210°.

Die nachstehenden Beispiele betreffen pharmazeutische Zubereitungen, die Verbindungen der Formel I oder ihre physiologisch unbedenklichen Salze enthalten:

### Beispiel A Tabletten

Ein Gemisch von 1 kg 2,2-Dimethyl-4-(1H-2-pyridon-1-yl)-6-aza-2H-chromen, 400 kg Lactose, 120 kg Kartoffelstärke, 20 kg Talk und 10 kg Magnesiumstearat wird in üblicher Weise zu Tabletten gepreßt, derart, daß jede Tablette 1 mg Wirkstoff enthält.

### Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C Kapseln

Man füllt 1 kg 2,2-Dimethyl-4-(1H-2-pyridon-1-yl)-6-aza-chroman-3-ol in üblicher Weise in Hartgelatinekapseln, so daß jede Kapsel 2 mg Wirkstoff enthält.

### Beispiel D Ampullen

Eine Lösung von 1 kg 2,2-Dimethyl-4-(1H-2-pyridon-1-yl)-7-aza-2H-chromen in 1000 l zweifach destilliertem Wasser wird steril filtriert, in Ampullen abgefüllt und steril verschlossen. Jede Ampulle enthält 1 mg Wirkstoff.

### Beispiel E Lösung zur topischen Anwendung (gegen Alopezie)

Man löst 500 g "A" (oder "B") in einem Gemisch von 5,2 kg 1,2-Propandiol und 15 l Ethanol, füllt mit Ethanol auf 25 l auf, filtriert steril und füllt in Flaschen.

### Beispiel F Gel

Man mischt 0,45 g Carbopol 934 P (= Carboxyvinyl-polymer) mit 40 ml zweifach destilliertem Wasser und 27 ml Ethanol, gibt eine Lösung von 0,5 g "A" (oder "B") und 0,45 g Diisopropanolamin in 10 ml 1,2-Propandiol und 13 ml Ethanol hinzu, mischt gut durch, füllt mit Wasser auf 100 ml auf, und mischt erneut gut durch. Das erhaltene Gel enthält 0,5 Gew.% Wirkstoff.

Analog sind Tabletten, Dragees, Kapseln, Ampullen, Lösungen oder Gele erhältlich, die einen oder mehrere der übrigen Wirkstoffe der Formel I und/oder ihrer physiologisch unbedenklichen Salze enthalten.

## Patentansprüche (Patentansprüche für folgende(n) Vertragsstaat(en): AT, BE, CH, DE, FR, GB, IT, LI, NL, SE)

1. Aza-chromanderivate der Formel I 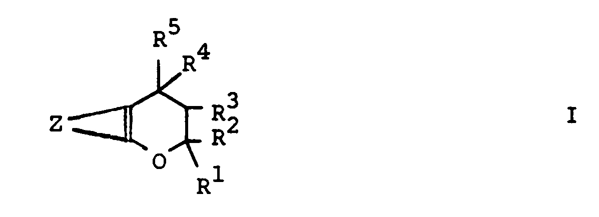 worin
Z (a) -N=CH-CH=CH-,
(b) -CH=N-CH=CH-,
(c) -CH=CH-N=CH- oder
(d) -CH=CH-CH=N-,
R¹ A,
R² H oder A,
R¹ und R² zusammen auch Alkylen mit 3-6 C-Atomen,
R³ OH oder OAc,
R⁴ H,
R³ und R⁴ zusammen auch eine Bindung,
R⁵ einen unsubstituierten oder ein- oder zweifach durch A, F, Cl, Br, J, OH, OA, OAc, NO₂, NH₂, AcNH, HOOC und/oder AOOC substituierten 1H-2-Pyridon-1-yl)-, 1H-6-Pyridazinon-1-yl)-, 1H-2-Pyrimidinon-1-yl)-, 1H-6-Pyrimidinon-1-yl)-, 1H-2-Pyrazinon-1-yl)- oder 1H-2-Thiopyridon-1-yl)-rest, wobei diese Reste auch partiell hydriert sein können,
A Alkyl mit 1-6 C-Atomen und
Ac Alkanoyl mit 1-8 C-Atomen oder Aroyl mit 7-11 C-Atomen
bedeuten,
sowie deren Salze.

2. a) 2,2-Dimethyl-4-(1H-2-pyridon-1-yl)-5-aza-chroman-3-ol;
b) 2,2-Dimethyl-4-(1H-2-pyridon-1-yl)-5-aza-2H-chromen;
c) 2,2-Dimethyl-4-(1H-2-pyridon-1-yl)-6-aza-chroman-3-ol;
d) 2,2-Dimethyl-4-(1H-2-pyridon-1-yl)-6-aza-2H-chromen;
e) 2,2-Dimethyl-4-(1H-2-pyridon-1-yl)-7-aza-chroman-3-ol;
f) 2,2-Dimethyl-4-(1H-2-pyridon-1-yl)-7-aza-2H-chromen;
g) 2,2-Dimethyl-4-(1H-2-pyridon-1-yl)-8-aza-chroman-3-ol;
h) 2,2-Dimethyl-4-(1H-2-pyridon-1-yl)-8-aza-2H-chromen.

3. Verfahren zur Herstellung von Aza-chromanderivaten der Formel I, dadurch gekennzeichent, daß man ein 3,4-Epoxy-aza-chroman der Formel II 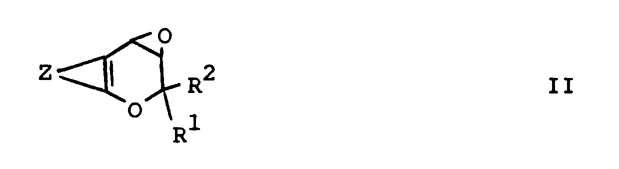 xworin
R¹, R² und Z die bei Formel I angegebene Bedeutung haben,
mit einer Verbindung der Formel III
R⁵-H III
worin R⁵ die bei Formel I angegebene Bedeutung hat oder mit einem ihrer reaktionsfähigen Derivate umsetzt
oder daß man ein sonst der Formel I entsprechendes N-Oxid reduziert und/oder daß man eine Verbindung der Formel I, worin R³ OH und R⁴ H bedeutet, dehydratisiert und/oder daß man in einer Verbindung der Formel I einen oder mehrere der Reste R³ und/oder R⁵ in andere Reste R³ und/oder R⁵ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

## Patentansprüche (Patentansprüche für folgende(n) Vertragsstaat(en): ES)

1. Verfahren zur Herstellung von Aza-chromanderivaten der Formel I 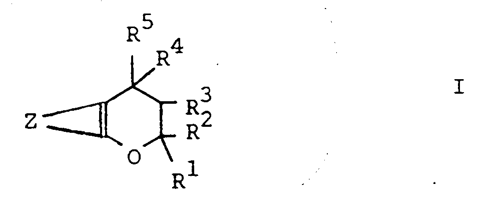 worin
Z (a) -N=CH-CH=CH-,
(b) -CH=N-CH=CH-,
(c) -CH=CH-N=CH- oder
(d) -CH=CH-CH=N-,
R¹ A,
R² H oder A,
R¹ und R² zusammen auch Alkylen mit 3-6 C-Atomen,
R³ OH oder OAc,
R⁴ H,
R³ und R⁴ zusammen auch eine Bindung,
R⁵ einen unsubstituierten oder ein- oder zweifach durch A, F, Cl, Br, J, OH, OA, OAc, NO₂, NH₂, AcNH, HOOC und/oder AOOC substituierten 1H-2-Pyridon-1-yl-, 1H-6-Pyridazinon-1-yl-, 1H-2-Pyrimidinon-1-yl-, 1H-6-Pyrimidinon-1-yl-, 1H-2-Pyrazinon-1-yl- oder 1H-2-Thiopyridon-1-yl-rest, wobei diese Reste auch partiell hydriert sein können,
A Alkyl mit 1-6 C-Atomen und
Ac Alkanoyl mit 1-8 C-Atomen oder Aroyl mit 7-11 C-Atomen
bedeuten,
sowie von deren Salzen, dadurch gekennzeichnet, daß man ein 3,4-Epoxy-aza-chroman der Formel II 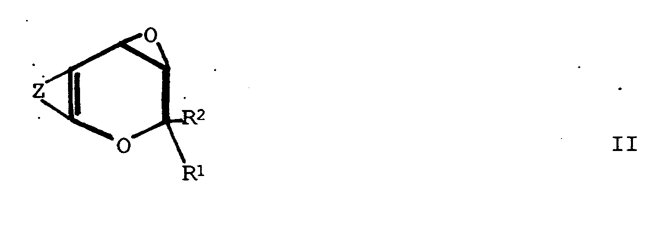 worin
R¹, R² und Z die bei Formel I angegebene Bedeutung haben, mit einer Verbindung der Formel III
R⁵-H III
wobei R⁵ die bei Formel I angegebene Bedeutung hat oder mit einem ihrer reaktionsfähigen Derivate umsetzt
oder daß man ein sonst der Formel I entsprechendes N-Oxid reduziert und/oder daß man eine Verbindung der Formel I, worin R³ OH und R⁴ H bedeutet, dehydratisiert und/oder daß man in einer Verbindung der Formel I einen oder mehrere der Reste R³ und/oder R⁵ in andere Reste R³ und/oder R⁵ umwandelt und/oder daß man eine basische Verbindung der Formel I durch Behandeln mit einer Säure in eines ihrer Säureadditionssalze umwandelt.

2. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

## Claims (Claims for the following Contracting State(s): AT, BE, CH, DE, FR, GB, IT, LI, NL, SE)

1. Azachroman derivatives of the formula I 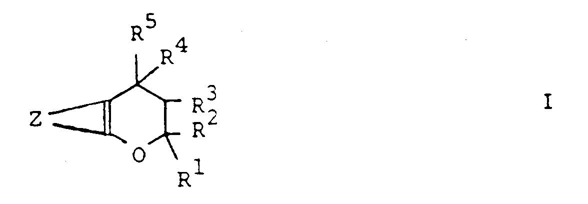 wherein
Z is (a) -N=CH-CH=CH-,
(b) -CH=N-CH=CH-,
(c) -CH=CH-N=CH- or
(d) -CH=CH-CH=N-,
R¹ is A
R² is H or A,
R¹ and R² together are also alkylene with 3-6 C atoms,
R³ is OH or OAc,
R⁴ is H,
R³ and R⁴ together are also a bond,
R⁵ is a 1H-2-pyridon-1-yl, 1H-6-pyridazinon-1-yl, 1H-2-pyrimidinon-1-yl, 1H-6-pyrimidinon-1-yl, 1H-2-pyrazinon-1-yl or 1H-2-thiopyridon-1-yl radical which is unsubstituted or mono- or di-substituted by A, F, Cl, Br, I, OH, OA, OAc, NO₂, NH₂, AcNH, HOOC and/or AOOC, it also being possible for these radicals to be partly hydrogenated,
A is alkyl with 1-6 C atoms and
Ac is alkanoyl with 1-8 C atoms or aroyl with 7-11 C atoms,
and salts thereof.

2. a) 2,2-Dimethyl-4-(1H-2-pyridon-1-yl)-5-azachroman-3-ol;
b) 2,2-Dimethyl-4-(1H-2-pyridon-1-yl)-5-aza-2H-chromene;
c) 2,2-Dimethyl-4-(1H-2-pyridon-1-yl)-6-azachroman-3-ol;
d) 2,2-Dimethyl-4-(1H-2-pyridon-1-yl)-6-aza-2H-chromene;
e) 2,2-Dimethyl-4-(1H-2-pyridon-1-yl)-7-azachroman-3-ol;
f) 2,2-Dimethyl-4-(1H-2-pyridon-1-yl)-7-aza-2H-chromene;
g) 2,2-Dimethyl-4-(1H-2-pyridon-1-yl)-8-azachroman-3-ol;
h) 2,2-Dimethyl-4-(1H-2-pyridon-1-yl)-8-aza-2H-chromene.

3. Process for the preparation of azachroman derivatives of the formula I, characterised in that a 3,4-epoxy-azachroman of the formula II 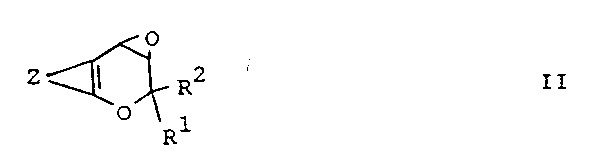 wherein
R¹, R² and Z have the meaning given in the case of formula I, is reacted with a compound of the formula III
R⁵-H III
wherein R⁵ has the meaning given in the case of formula I, or with one of its reactive derivatives, or in that an N-oxide otherwise corresponding to the formula I is reduced and/or in that a compound of the formula I wherein R³ is OH and R⁴ is H is dehydrated and/or in that one or more of the radicals R³ and/or R⁵ in a compound of the formula I is/are converted into other radicals R³ and/or R⁵, and/or in that a basic compound of the formula I is converted into one of its acid addition salts by treatment with an acid.

4. Process for the preparation of pharmaceutical formulations, characterised in that a compound of the formula I and/or one of its physiologically acceptable salts is brought into a suitable dosage form together with at least one solid, liquid or semi-liquid excipient or auxiliary and if appropriate in combination with one of more other active compound(s).

5. Pharmaceutical formulation, characterised in that it contains at least one compound of the formula I and/or one of its physiologically acceptable salts.

6. Compounds of the formula I for combating diseases.

7. Use of compounds of the formula I for the preparation of a medicament.

## Claims (Claims for the following Contracting State(s): ES)

1. Process for the preparation of azachroman derivatives of the formula I 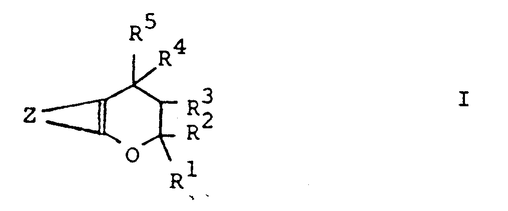 wherein
Z is (a) -N=CH-CH=CH-,
(b) -CH=N-CH=CH-,
(c) -CH=CH-N=CH- or
(d) -CH=CH-CH=N-,
R¹ is A,
R² is H or A,
R¹ and R² together are also alkylene with 3-6 C atoms,
R³ is OH or OAc,
R⁴ is H,
R³ and R⁴ together are also a bond,
R⁵ is a 1H-2-pyridon-1-yl, 1H-6-pyridazinon-1-yl, 1H-2-pyrimidinon-1-yl, 1H-6-pyrimidinon-1-yl, 1H-2-pyrazinon-1-yl or 1H-2-thiopyridon-1-yl radical which is unsubstituted or mono- or di-substituted by A, F, Cl, Br, I, OH, OA, OAc, NO₂, NH₂, AcNH, HOOC and/or AOOC, it also being possible for these radicals to be partly hydrogenated,
A is alkyl with 1-6 C atoms and
Ac is alkanoyl with 1-8 C atoms or aroyl with 7-11 C atoms,
and of salts thereof
characterised in that a 3,4-epoxy-azachroman of the formula II 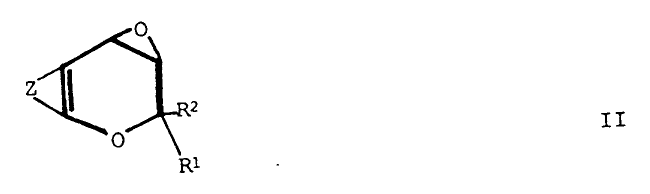 wherein
R¹, R² and Z have the meaning given in the case of formula I, is reacted with a compound of the formula III
R⁵-H III
wherein R⁵ has the meaning given in the case of formula I, or with one of its reactive derivatives, or in that an N-oxide otherwise corresponding to the formula I is reduced and/or in that a compound of the formula I wherein R³ is OH and R⁴ is H is dehydrated and/or in that one or more of the radicals R³ and/or R⁵ in a compound of the formula I is/are converted into other radicals R³ and/or R⁵, and/or in that a basic compound of the formula I is converted into one of its acid addition salts by treatment with an acid.

2. Process for the preparation of pharmaceutical formulations, characterised in that a compound of the formula I and/or one of its physiologically acceptable salts is brought into a suitable dosage form together with at least one solid, liquid or semi-liquid excipient or auxiliary and if appropriate in combination with one or more other active compound(s).

## Revendications (Revendications pour l'(les) Etat(s) contractant(s) suivant(s): AT, BE, CH, DE, FR, GB, IT, LI, NL, SE)

1. Dérivés d'azachromanne de formule I 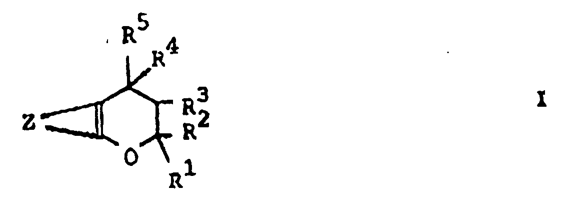 dans laquelle
Z désigne
(a) -N=CH-CH=CH-,
(b) -CH=N-CH=CH-,
(c) -CH=CH-N=CH- ou
(d) -CH=CH-CH=N-,
R¹ désigne A,
R² désigne H ou A,
R¹ et R² désignant également ensemble un radical alkylène avec 3 à 6 atomes C,
R³ désigne OH ou OAc,
R⁴ désigne H,
R³ et R⁴ désignant également ensemble une liaison,
R⁵ désigne un radical 1H-2-pyridon-1-yle, 1H-6-pyridazinon-1-yle, 1H-2-pyrimidinon-1-yle, 1H-6-pyrimidinon-1-yle, 1H-2-pyrazinon-1-yle ou 1H-2-thiopyridon-1-yle non substitués ou substitués une ou deux fois par A, F, Cl, Br, I, OH, OA, OAc, NO₂, NH₂, AcNH, HOOC et/ou AOOC, ces radicaux pouvant aussi être partiellement hydrogénés,
A désigne un radical alkyle avec 1 à 6 atomes C et
Ac désigne un radical alcanoyle avec 1 à 8 atomes C ou un radical aroyle avec 7 à 11 atomes C,
ainsi que leurs sels.

2. a) 2,2-diméthyl-4-(1H-2-pyridon-1-yl)-5-aza-chroman-3-ol;
b) 2,2-diméthyl-4-(1H-2-pyridon-1-yl)-5-aza-2H-chromène;
c) 2,2-diméthyl-4-(1H-2-pyridon-1-yl)-6-aza-chroman-3-ol;
d) 2,2-diméthyl-4-(1H-2-pyridon-1-yl)-6-aza-2H-chromène;
e) 2,2-diméthyl-4-(1H-2-pyridon-1-yl)-7-aza-chroman-3-ol;
f) 2,2-diméthyl-4-(1H-2-pyridon-1-yl)-7-aza-2H-chromène;
g) 2,2-diméthyl-4-(1H-2-pyridon-1-yl)-8-aza-chroman-3-ol;
h) 2,2-diméthyl-4-(1H-2-pyridon-1-yl)-8-aza-2H-chromène.

3. Procédé de préparation de dérivés d'aza-chromanne de formule I, caractérisé en ce qu'un 3,4-époxy-aza-chromanne de formule II 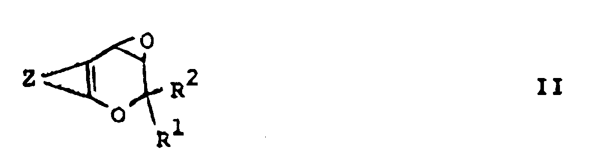 dans laquelle
R¹, R² et Z ont la signification mentionné à la formule I
est amené à réagir avec un composé de formule III
R⁵ - H III
dans laquelle R⁵ a la signification mentionnée à la formule I ou avec un de ses dérivés réactifs
ou en ce qu'un oxyde d'azote correspondant sinon à la formule I est réduit et/ou en ce qu'un composé de formule I dans laquelle R³ désigne OH et R⁴ désigne H est déshydraté et/ou en ce que, dans un composé de formule I, un ou plusieurs des radicaux R³ et/ou R⁵ sont transformés en d'autres radicaux R³ et/ou R⁵ et/ou en ce qu'un composé basique de formule I est transformé par traitement avec un acide en son sel d'acide.

4. Procédé de fabrication de préparations pharmaceutiques, caractérisé en ce qu'un composé de formule I et/ou un de ses sels physiologiquement neutres est amené dans une forme de dosage adéquate avec au moins un support ou un adjuvant solide, liquide ou semi-liquide et, éventuellement, en combinaison avec une ou plusieurs autres substances actives.

5. Préparation pharmaceutique, caractérisée par une teneur en au moins un composé de formule I et/ou un de ses sels physiologiquement neutres.

6. Composé de formule I de lutte contre les maladies.

7. Mise en oeuvre de composée de formule I pour la préparation d'un médicament.

## Revendications (Revendications pour l'(les) Etat(s) contractant(s) suivant(s): ES)

1. Procédé de préparation de dérivés d'aza-chromanne de formule I 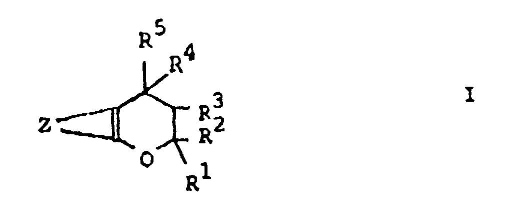 dans laquelle
Z désigne
(a) -N=CH-CH=CH-,
(b) -CH=N-CH=CH-,
(c) -CH=CH-N=CH- ou
(d) -CH=CH-CH=N-,
R¹ désigne A,
R² désigne H ou A,
R¹ et R² désignent également ensemble un radical alkylène avec 3 à 6 atomes C,
R³ désigne OH ou OAc,
R⁴ désigne H,
R³ et R⁴ désignent également ensemble une liaison,
R⁵ désigne un radical 1H-2-pyridon-1-yle, 1H-6-pyridazinon-1-yle, 1H-2-pyrimidinon-1-yle, 1H-6-pyrimidinon-1-yle, 1H-2-pyrazinon-1-yle ou 1H-2-thiopyridon-1-yle non substitués ou substitués une ou deux fois par A, F, Cl, Br, I, OH, OA, OAc, NO₂, NH₂, AcNH, HOOC et/ou AOOC, ces radicaux pouvant aussi être partiellement hydrogénés,
A désigne un radical alkyle avec 1 à 6 atomes C et
Ac désigne un radical alcanoyle avec 1 à 8 atomes C ou un radical aroyle avec 7 à 11 atomes C,
ainsi que leurs sels, caractérisé en ce qu'un 3,4-époxy-aza-chromanne de formule II 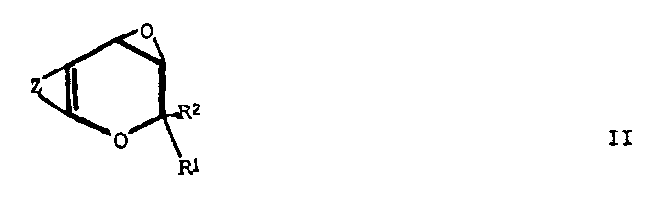 dans laquelle
R¹, R² et Z ont la signification mentionnée à la formule I
est amené à réagir avec un composé de formule III
R⁵ - H III
dans laquelle R⁵ a la signification mentionnée à la formule I ou avec un de ses dérivés réactifs
ou on ce qu'un oxyde d'azote correspondant sinon à la formule I est réduit et/ou en ce qu'un composé de formule I dans laquelle R³ désigne OH et R⁴ désigne H est déshydraté et/ou en ce que, dans un composé de formule I, un ou plusieurs des radicaux R³ et/ou R⁵ sont transformés en d'autres radicaux R³ et/ou R⁵ et/ou en ce qu'un composé basique de formule I est transformé par traitement avec un acide en son sel d'acide.

2. Procédé de fabrication de préparations pharmaceutiques, caractérisé en ce qu'un composé de formule I et/ou un de ses sels physiologiquement neutres est amené dans une forme de dosage adéquate avec au moins un support ou un adjuvant solide, liquide ou semi-liquide et, éventuellement, en combinaison avec une ou plusieurs autres substances actives.
